# EUROPEAN PATENT APPLICATION

(11) **EP 4 332 579 A1**
(43) Date of publication of application: **06.03.2024**
(21) Application number: 22795205.8
(22) Date of filing: 02.02.2022
(51) Int. Cl.: G01N 35/00, G01N 30/86

(54) **AUTOMATIC ANALYSIS DEVICE**

(30) Priority: 27.04.2021 JP 2021074742
(71) Applicant: HITACHI HIGH-TECH CORPORATION, Tokyo 105-6409 (JP)
(72) Inventor: NOGAMI, Makoto, Tokyo 105-6409 (JP); TAMURA, Riku, Tokyo 105-6409 (JP); NISHIKI, Kenichiro, Tokyo 105-6409 (JP); SUGIME, Takayuki, Tokyo 105-6409 (JP); HASHIMOTO, Yuichiro, Tokyo 105-6409 (JP); KATANO, Toshiaki, Tokyo 105-6409 (JP); SUGIYAMA, Masuyuki, Tokyo 100-8280 (JP)
(74) Representative: Strehl Schübel-Hopf & Partner
(86) International application number: PCT/JP2022/004104
(87) International publication number: WO 2022/230282

(57) **Abstract**

To provide an automatic analyzer capable of quickly responding when an abnormal process occurs, accurately grasping an abnormal state, and reducing frequencies of early abnormality diagnosis and component adjustment or replacement of the automatic analyzer. The automatic analyzer compares data output information with reference data output information, applies a data alarm to an item of the data output information determined to be in an abnormal state, compares system output information with reference system output information, applies a data alarm to an item of a data output information determined to be in an abnormal state, and executes a predictive diagnosis of a failure state of the automatic analyzer based on a temporal change of the data output information obtained by the analyzer using an internal standard substance as a sample and a temporal change of the system output information. The data output information includes at least one item generated based on a detection result of the sample of the analyzer, and the system output information includes at least one item generated based on a detection result from a sensor provided in the analyzer.

## Description

### Technical Field

The present invention relates to an automatic analyzer.

### Background Art

A liquid chromatograph mass spectrometer (HPLC/MS) has been known as an automatic analyzer. Specifically, the HPLC/MS is configured to perform qualification/quantification of the respective components in the sample by combining a separation process executed by the liquid chromatograph (HPLC) based on the chemical structure and physical properties of a substance to be measured, and a separation process executed by the mass spectrometer (MS) based on the mass of the substance to be measured.

The liquid chromatograph mass spectrometer (HPLC/MS) is capable of performing qualification/quantification of the substance to be measured even in the case of co-existence of many analogous substances metabolized in vivo like a pharmaceutical product in the biological sample. Accordingly, application of the HPLC/MS to the clinical examination field has been expected. In the examination center, university hospital, and the like, the liquid chromatograph mass spectrometer (HPLC/MS) has been used for examining an immunosuppressant, an anticancer agent, a neonate metabolic disorder testing, a TDM (Therapeutic Drug Monitoring), and the like.

In the liquid chromatograph mass spectrometer, execution of the complicated preprocessing step leads to examination results that may vary depending on the skill level of the test engineer. When performing the preprocessing and measurement by the liquid chromatograph mass spectrometer (HPLC/MS), a human error may lead to incorrectness of the examination result. It has been demanded to develop the automatic analyzer capable of executing fully automatic batch processing from the preprocessing to the operation of the liquid chromatograph mass spectrometer (HPLC/MS) towards the clinical examination field.

Meanwhile, the automatic analyzer is required to assure robustness of the device by diagnosing the operation abnormality and the failure state at an earlier stage, or performing the prediction at the earlier stage to execute the predictive diagnosis for the maintenance work such as adjustment and replacement of the automatic analyzer and the component. Execution of the predictive diagnosis secures to minimize a sudden stop of operations owing to failure and defect of the device or the component, financial damages owing to the mechanical loss, or damages to health and life of the operator or the user of the device. The following technique has been known as the predictive diagnosis executed by the automatic analyzer.

Patent Literature 1 discloses a clinical diagnosis system (100) which includes a sample preparation module (20) for automatically preparing the sample, a liquid chromatography (LC) separation module (30) to be combined with the sample preparation module (20) by a sample preparation/LC interface (40), a mass spectrometer (MS) module (50) to be combined with the LC separation module (30) by an LC/MS interface (60), a result calculation module (70) for identification and/or quantification of a target analyte or a target substance which is contained in the sample, and caused to pass through the LC separation module (30) and the MS module (50), and a control unit (80). The control unit (80) monitors a predetermined set of operation parameters (1 to n) each indicating a performance state of the clinical diagnosis system (100). Upon every deviation of one or more parameters (1 to n) of the predetermined set of the operation parameters (1 to n) from the specification, the control unit is programmed to activate the quality control process and/or the maintenance process. The control unit is further programmed to minimize the quality control process and/or the maintenance process so long as the set of the operation parameters (1 to n) is kept within the specification.

Patent Literature 2 discloses a clinical sample processing device which can be connected to an externally provided maintenance managing device via a network. The clinical sample processing device includes a sample processing unit for processing the sample by causing the respective mechanisms to perform predetermined operations, a comparison unit for comparing a numerical value based on an operation detection signal indicating a predetermined operation of each mechanism with a threshold value as a reference based on which it is determined whether or not the predetermined operation is in a normal state, and a transmission unit. When the numerical value based on the operation detection signal of the predetermined operation of any one of the multiple mechanisms exceeds the threshold value, the transmission unit transmits failure predication information indicating a higher possibility of failure of the mechanism in the future than the one in the normal state to the maintenance managing device via the network.

### Citation List

### Patent Literature

Patent Literature 1: WO 2019/007868
Patent Literature 2: Japanese Unexamined Patent Application Publication No. 2008-89616

### Summary of Invention

### Technical Problem

In the prior art as disclosed in Patent Literature 1, based on the QC and the maintenance operations upon deviation of one or more predetermined parameters from the specification as a trigger, the program for monitoring a set of one or more predetermined operation parameters is controlled to minimize the QC and the maintenance operations to be within the specification upon deviation of one or more predetermined parameters from the specification. As the QC and the maintenance operations are set as the trigger, the measurement using the sample has to be performed to monitor the set of the predetermined operation parameters. As the program is designed to bring those parameters to be within the specification for each measurement, the temporal change information across the measurements cannot be processed.

The prior art disclosed in Patent Literature 2 includes the comparison unit which compares the numerical value based on the operation detection signal indicating the predetermined operation of each mechanism with the threshold value as the reference for determination whether or not the predetermined operation is in the normal state. If the numerical value based on the operation detection signal indicating the predetermined operation of any one of the mechanisms exceeds the threshold value, failure prediction information is output for indicating the higher possibility of failure of the mechanism in the future than the one in the normal state. Based on the numerical value of the signal output by the sensor in the device, and the processing completion time, the device outputs signals to the maintenance management device based on the two-step determination criteria. Specifically, when the value exceeds the first threshold value, a "warning" is transmitted. When the value further exceeds the second threshold value, an "abnormal state" is transmitted. The value output from each sensor in the device is used as a determination factor with respect to the "warning" or "abnormal state" without using the information originated from the output value of data from the detection unit (MS) as the determination factor. Accordingly, the failure prediction accuracy needs to be further improved. The temporal change information across the measurements cannot be processed.

The present invention has been made in light of the above-described circumstances. It is an object of the present invention to provide an automatic analyzer capable of quickly responding to occurrence of an abnormal process, accurately grasping an abnormal state, and reducing frequencies of early abnormality diagnosis and component adjustment or replacement of the device. Solution to Problem

The present invention provides a plurality of measures for solving the above-described problems. For example, an automatic analyzer includes an analyzer having a separation unit configured to perform separation of a sample to be measured subjected to a pretreatment, and a detection unit configured to detect a substance to be measured of the sample separated by the separation unit, and a control device configured to control an operation of the analyzer. The control device includes a data alarm database that stores data output information including at least one item generated based on a detection result of the sample of the analyzer, a normal data alarm database that stores predetermined reference data output information to determine whether the data output information is in a normal state or in an abnormal state, a data alarm determination unit configured to compare the data output information of the data alarm database with the reference data output information of the normal data alarm database and apply a data alarm to an item of the data output information of the data alarm database determined to be in an abnormal state, a system alarm database that stores system output information including at least one item generated based on a detection result from a sensor provided in the analyzer, a normal system alarm database that stores predetermined reference system output information to determine whether the system output information is in a normal state or in an abnormal state, and a system alarm determination unit configured to compare the system output information of the system alarm database with the reference system output information of the normal system alarm database and apply a data alarm to an item of the data output information of the system alarm database determined to be in an abnormal state. The control device is configured to execute a predictive diagnosis of a failure state of the automatic analyzer based on a temporal change of the data output information that is obtained by the analyzer using an internal standard substance as the sample and is stored in the data alarm database and a temporal change of the system output information of the system alarm database.

### Advantageous Effects of Invention

The present invention allows quick response to occurrence of an abnormal process, accurate grasping of an abnormal state, and reduction in frequencies of early abnormality diagnosis and component adjustment or replacement of the device.

### Brief Description of Drawings

[FIG. 1] FIG. 1 is a functional block diagram schematically showing an overall configuration of an automatic analysis system.
[FIG. 2] FIG. 2 is a top view schematically showing an example of a configuration of a preprocessing unit.
[FIG. 3] FIG. 3 is a diagram schematically showing an example of a configuration of a separation unit.
[FIG. 4] FIG. 4 is a diagram schematically showing an example of a configuration of a detection unit.
[FIG. 5] FIG. 5 is a flowchart showing contents of an analysis operation of an automatic analyzer.
[FIG. 6] FIG. 6 is a diagram showing an example of a display screen displayed on a display device.
[FIG. 7] FIG. 7 is a diagram showing an example of the display screen displayed on the display device.
[FIG. 8] FIG. 8 is a diagram showing an example of the display screen displayed on the display device.

### Description of Embodiments

One embodiment of the invention will now be described with reference to the drawings.

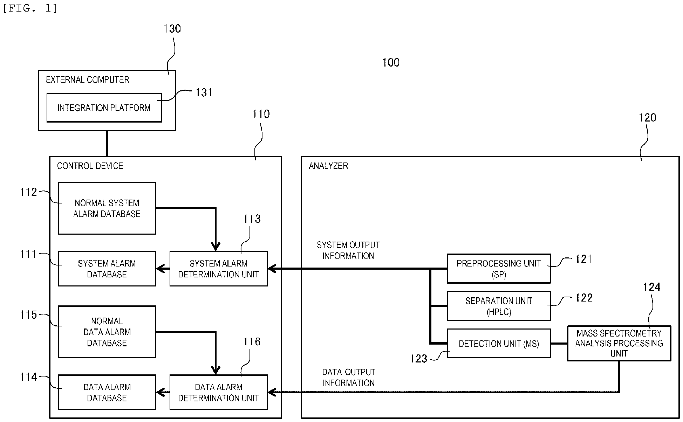

FIG. 1 is a functional block diagram schematically showing an overall configuration of an automatic analyzer according to an embodiment.

Referring to FIG. 1, an automatic analyzer 100 mainly includes a control device 110, an analyzer 120, and an external computer 130.

The analyzer 120 includes a preprocessing unit (SP) 121, a separation u nit (HPLC) 122, a detection unit (MS) 123, and a mass spectrometry analysis processing unit 124.

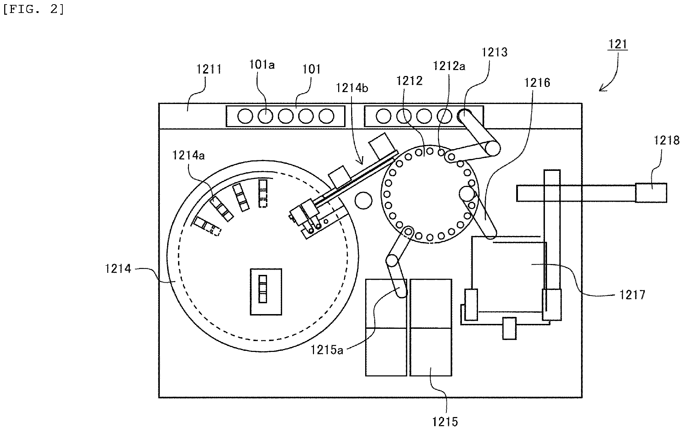

FIG. 2 is a top view schematically showing an example of a configuration of a preprocessing unit.

Referring to FIG. 2, the preprocessing unit (SP) 121 includes a sample transport unit 1211 which transports a sample container rack 101 having multiple sample containers 101a each storing a sample or a specimen to be measured mounted thereon to a dispensation position, a reaction disk unit 1212 provided with multiple reaction containers 1212a where a magnetic bead and a reagent are added to the sample which contains the substance to be measured for reaction, a sample probe 1213 which dispenses (aspires/discharges) the sample in the sample container 101a on the sample container rack 101 which has been transported to the dispensation position to the reaction container 1212a of the reaction disk unit 1212, a reagent disk unit 1214 which holds a reagent container 1214a for storing the magnetic bead and the reagent, a reagent dispensing unit 1214b which dispenses (aspires/discharges) the magnetic bead and the reagent to the reaction disk unit 1212, a reagent container holding unit 1215 which holds the reagent container, a reagent probe 1215a which dispenses (aspires/discharges) the reagent of the reagent container holding unit 1215 to the reaction disk unit 1212, a cleaning unit 1217 which cleans the magnetic bead transferred with the sample containing the substance to be measured for reaction in the reaction container 1212a of the reaction disk unit 1212, a washing probe 1216 which dispenses (aspires/discharges) the sample and the magnetic bead from the reaction disk unit 1212 to the cleaning unit 1217, and a separation unit transport unit 1218 which transports the sample to the separation unit (HPLC) 122.

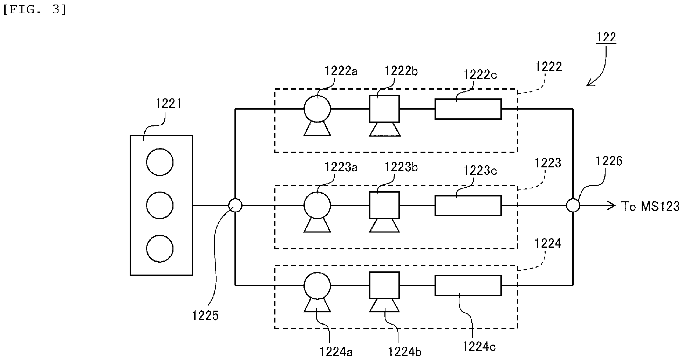

FIG. 3 is a diagram schematically showing an example of a configuration of a separation unit.

The separation unit (HPLC) 122 has multiple streams (three streams in the embodiment, for example), that is, HPLC1 stream 1222, HPLC2 stream 1223, HPLC3 stream 1224 for improving throughput, and includes an input side stream select valve 1225 positioned in a preceding stage of the respective streams for switching among the streams, and an output side stream select valve 1226 positioned in a subsequent stage of the respective streams for switching among the streams and connection to the detection unit (MS) 123 in the subsequent stage of the respective streams.

The streams 1222, 1223, 1224 include pump units 1222a, 1223a, 1224a for feeding solvents and reagents to a flow passage, sample introduction units 1222b, 1223b, 1224b for introducing the sample transferred from the preprocessing unit (SP) 121 to the flow passage, and separation columns 1222c, 1223c, 1224c for separating the sample, respectively. A system reagent unit 1221 capable of selecting the reagent to be used from multiple reagents is provided at the upstream side of the input side stream select valve 1225.

Each of the pump units 1222a, 1223a, 1224a has two cylinders driven to maintain the set flow rate, an auto purge valve for switching between the flow passage and the waste liquid to remove air in the flow passage, and a pump pressure sensor for monitoring pressure in the flow passage.

Each of the sample introduction units 1222b, 1223b, 1224b includes an injection valve provided in each stream for switching between a liquid feed passage from the pump and the flow passage from the syringe, a sample loop connected to the injection valve, a syringe for aspirating/discharging the sample, and a syringe pressure sensor for monitoring the aspiration/discharging state of the syringe. The sample transported from the preprocessing unit (SP) 121 is transported to a sample aspiration position at each stream in the separation unit transport unit 1218. After aspiration, the sample is transported to a waste liquid aspiration position common to the respective streams so that the solution remained in the sample container is aspired/discharged by a waste liquid probe.

The separation columns 1222c, 1223c, 1224c are held in column cartridges, respectively each provided with a heat conduction block at a lower part. The column cartridge has its temperature controlled in a column oven having a heater, a heat block and a thermistor for sensing the temperature. The column cartridge is fixed at a given position in the column oven, and monitored by a position sensor.

The system reagent unit 1221 includes a lifter for mounting each reagent bottle onto a normal position, and a weight sensor for monitoring an amount of the reagent in each reagent bottle. The system reagent unit 1221 includes a lifter for mounting the reagent bottle, and the weight sensor for monitoring an amount of the reagent in the reagent bottle.

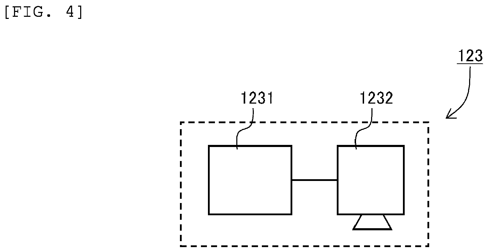

FIG. 4 is a diagram schematically showing an example of a configuration of a detection unit.

The detection unit (MS) 123 includes an ion source unit 1231 and a mass spectrometer 1232.

Data generated by the mass spectrometer 1232, and the calculation method will be described. The mass spectrometer 1232 generates data of signal intensity (area) having a specific m/z (mass/charge number) counted by the detector. In the preprocessing unit (SP) 121, an internal standard substance is added to the sample which contains the substance to be measured to generate the data of signal intensities corresponding to the m/z of the substance to be measured and the m/z of the internal standard substance. The mass spectrometer 1232 performs measurement of the known internal standard substance added to several substances to be measured at different known concentrations. A calibration curve is then prepared preliminarily by plotting the concentration of the substance to be measured on the horizontal axis, and the ratio of the signal intensity (area) corresponding to those of the substance to be measured and the internal standard substance on the vertical axis. The concentration of the substance to be measured is calculated from the preliminarily prepared calibration curve, and the ratio of the signal intensity (area) corresponding to data of those to the m/z of the substance to be measured and the m/z of the internal standard substance, both of which have been generated by the mass spectrometer 1232.

An output of the detection unit (MS) 123 is transmitted to the mass spectrometry analysis processing unit 124. A peak fitting is performed with reference to the signal intensity (area) information having the specific m/z (mass/charge number) counted by the detector to generate data output information. A function of the mass spectrometry analysis processing unit 124 is implemented using software which runs on the detection unit (MS) 123, for example.

### <Data output information>

Data output information generated by the mass spectrometer 1232 and mass spectrometry analysis processing unit 124 will be described. The data output information includes:
1) Peak area ratio of m/z for quantification of substrate to be measured and m/z for quantification of internal standard substance
2) Peak intensity ratio of m/z for quantification of substrate to be measured and m/z for quantification of internal standard substance
3) Peak area of m/z for quantification of substrate to be measured
4) Peak intensity of m/z for quantification of substrate to be measured
5) Retention time of m/z for quantification of substrate to be measured
6) Peak width of m/z for quantification of substrate to be measured
7) Full width at half maximum of m/z for quantification of substrate to be measured
8) S/N (ratio of peak intensity/background) of m/z for quantification of substance to be measured
9) Leading factor of m/z for quantification of substrate to be measured
10) Tailing factor of m/z for quantification of substrate to be measured
11) Peak area of m/z for qualification of substrate to be measured
12) Peak intensity of m/z for qualification of substrate to be measured
13) Retention time of m/z for qualification of substrate to be measured
14) Peak width of m/z for qualification of substrate to be measured
15) Full width at half maximum of m/z for qualification of substrate to be measured
16) S/N (ratio of peak intensity/background) of m/z for qualification of substance to be measured
17) Peak area of m/z for quantification of internal standard substance
18) Peak intensity of m/z for quantification of internal standard substance
19) Retention time m/z for quantification of internal standard substance
20) Peak width of m/z for quantification of internal standard substance
21) Full width at half maximum of m/z for quantification of internal standard substance
22) S/N (ratio of peak intensity/background) of m/z for quantification of internal standard substance
23) Leading factor of m/z for quantification of substrate to be measured
24) Tailing factor of m/z for quantification of substrate to be measured
25) Peak area of m/z for qualification of internal standard substance
26) Peak intensity of m/z for qualification of internal standard substance
27) Retention time of m/z for qualification of internal standard substance
28) Peak width of m/z for qualification of internal standard substance
29) Full width at half maximum of m/z for qualification of internal standard substance
30) S/N (ratio of peak intensity/background) of m/z for qualification of the internal standard substance

The data output information is not limited to those as described above.

Referring to FIG. 1, the control device 110 for controlling an operation of the analyzer 120, includes a data alarm database 114 which stores "data output information" generated by the analyzer 120, a normal data alarm database 115 which stores normal state data information, a data alarm determination unit 116, a system alarm database 111 which stores "system output information" generated by the analyzer 120, a normal system alarm database 112 which stores normal state system alarm information, and a system alarm determination unit 113.

The data output information from the analyzer 120 is transferred to the control device 110, and compared with the normal data alarm database 115 by the data alarm determination unit 116. The data output information deviating from the threshold value is determined as an abnormal value, and stored in the data alarm database 114. The user is notified of the data alarm affixed to the examination result. At this time, the user confirms the data to determine whether the re-examination is necessary. The data alarm may have a specific item to be subjected to automatic re-measurement.

The system output information from the analyzer 120 is transferred to the control device 110, and compared with the normal system alarm database 112 which stores the normal system alarm information by the system alarm determination unit 113. The system output information deviating from the threshold value is determined as an abnormal value, and stored in the system alarm database 111. The user is notified of the system alarm.

The external computer 130 accurately grasps the abnormal state from the information of the system alarm database 111 and the data alarm database 114, which has been determined as having abnormality by the control device 110. This makes it possible to perform early abnormality diagnosis of the device, and to determine execution of adjustment/replacement of components. The external computer 130 includes an integration platform 131 built by the user, and connected to clinical examination devices of various types to share the information relating to the early abnormality diagnosis of device, and adjustment/replacement of components.

### <Data alarm>

Determination items and the determination method to be implemented by the data alarm determination unit 116 will be described. The data alarm database 114 applies a data alarm to a determination item determined as being abnormal.
1) Internal standard substance signal check (lower limit value): If the peak intensity of the internal standard substance is equal to or lower than the lower limit value, an abnormal state is determined.
2) Internal standard substance signal check (upper limit value): If the peak intensity of the internal standard substance is equal to or higher than a maximum value, the abnormal state is determined.
3) Internal standard substance identification check: a peak parameter (for example, a retention time of the internal standard substance, a peak width, a full width at half maximum, an S/N (ratio of peak intensity/background)) is checked. If the peak parameter deviates from the range, the abnormal state is determined.
4) Substance to be measured signal check (lower limit value)
   If the peak intensity of the substance to be measured is equal to or lower than the lower limit value, an abnormal state is determined.
5) Substance to be measured signal check (upper limit value): If the peak intensity of the substance to be measured is equal to or higher than a maximum value, the abnormal state is determined.
6) Substance to be measured identification check: A peak parameter (for example, a retention time of the internal standard substance, a peak width, a full width at half maximum, an S/N (ratio of peak intensity/background)) is checked. In the case of deviation from the range, the abnormal state is determined.
7) HPLC performance check: A pressure curve of HPLC, an ESI spray current, a peak parameter (a peak width, a peak symmetry, a retention time, and the like) of the internal standard substance are checked. In the case of deviation from the range, the abnormal state is determined.
8) MS performance check: If the peak parameter, for example, the background intensity and/or the noise value deviates from the range, the abnormal state is determined. Contamination information is output as well.
9) Calibration curve check: The calibration curve is checked. In the case of deviation from the range, the abnormal state is determined.
10) QC check: The QC is checked. In the case of deviation from the range, the abnormal state is determined. The determination item and the determination method are not limited to those described above.

An analytic operation performed by the automatic analyzer 100 according to the embodiment will be described.

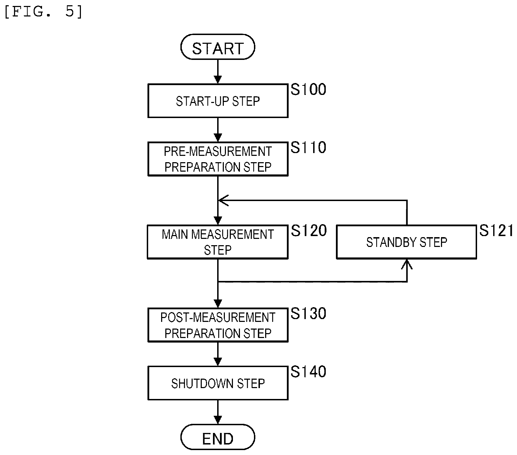

FIG. 5 is a flowchart showing contents of an analysis operation of the automatic analyzer.

As FIG. 5 represents, the automatic analyzer 100 executes the "start-up step" by turning power ON to activate the respective devices for confirming communication between the analyzer 120 and the control device 110, and each state of the sensors and consumables (step S100).

The "pre-measurement preparation step" is executed, in which a user or a service person selects the maintenance item, and conducts the selected maintenance (step S110). Upon completion of the pre-measurement preparation step, the "main measurement step" is executed for performing a calibration measurement, a QC measurement, and a sample measurement (step S120). After completion of the main measurement step, if the main measurement is continued intermittently, the "standby step" is executed for making the process standby until start of the next main measurement step (step S121).

When turning the automatic analyzer 100 OFF at the end of the main measurement in step S120, the "post-measurement preparation step" (step S130) is executed to prepare for turning the device OFF. The power is turned OFF to execute "shutdown step" for shutting down each device (step S140).

Explanations will be made with respect to the calibration measurement, QC measurement, and sample measurement, which are executed in the "main measurement step" (see step S120 in FIG. 5).

First, explanation will be made with respect to the calibration measurement. In the calibration measurement, signal intensity data to the m/z of the substance to be measured and the m/z of the internal standard substance are generated. The mass spectrometer measures several substances to be measured at different known concentrations each added with the known internal standard substance to preliminarily prepare the calibration curve by plotting the concentration of the substance to be measured on the horizontal axis, and the ratio of signal intensity (area) corresponding to each one of the substance to be measured and the internal standard substance on the vertical axis. The concentration of the substance to be measured is calculated from the preliminarily prepared calibration curve, and the ratio of the signal intensity (area) corresponding to data of signal intensity (area) to the m/z of the substance to be measured and the m/z of the internal standard substance, which have been generated by the mass spectrometer.

Next, explanation will be made with respect to the QC (Quality Control) measurement. The QC measurement is executed for management of accuracy, specifically, assuring that the measurement result exhibits correctness (accuracy), and the results of repetitive measurements hardly fluctuate (precision) so long as the sample is kept unchanged. The accuracy management is performed to assure that the measurement results are accurate, and to prove validity of the measurement method and the management method based on the measurement result record. Specifically, the QC measurement is performed using the QC sample at the known concentration. The concentration is calculated using the calibration curve of the calibration measurement having the generated result preliminarily acquired. It is evaluated whether the calculated concentration is within a specified value. In the series of the process steps, validation is made whether the calculated concentration value is effective.

Next, explanation will be made with respect to the sample measurement.

The sample measurement can be started after the effective state of the device has been confirmed by the calibration measurement and the QC measurement. In the sample measurement for data output information, the concentration of the substance to be measured is calculated from the calibration curve generated by the calibration measurement, and the ratio of signal intensity (area) corresponding to data of signal intensity (area) to the m/z of the substance to be measured and the m/z of the internal standard substance, which have been generated by the mass spectrometer. The data output information is transferred to the control device 110, and compared with the normal data alarm database 115 so that the data output information deviating from the threshold value is determined as an abnormal value, and stored in the data alarm database 114. The user is notified of the data alarm affixed to the examination result. The user confirms the data to determine whether the re-examination is necessary.

### <System output information>

An explanation will be made with respect to the system output information generated and output by the preprocessing unit (SP) 121, the separation unit (HPLC) 122, and the detection unit (MS) 123 of the analyzer 120. The automatic analyzer 100 executes detection of abnormality such as insufficient reagent, clogging with reagent, reading defect of RFID, and the like based on the information output by each sensor or software count from the start of measurement.

First, explanation will be made with respect to the system output information of the preprocessing unit (SP) 121. The system output information of the preprocessing unit (SP) 121 includes an aspiration position error owing to abnormality in the position sensor, which occurs during driving of the sample probe 1213, the reagent dispensing unit 1214b, and the washing probe 1216, and a clogging detection error owing to abnormality in the pressure sensor, which occurs during aspiration/discharging of the sample or the reagent. The information also includes an aspiration/discharging position error owing to abnormality in the position sensor, which occurs during driving of the sample transport unit 1211, the reaction disk unit 1212, the cleaning unit 1217, and the separation unit transport unit 1218, and a temperature abnormality error owing to abnormality in the temperature sensor of the reaction disk unit. If the above-described abnormality occurs, for example, abnormality in the position sensor of each probe, multiple alarms of different types are output, for example, the sample aspiration position, the sample discharging position, the original point position, the position deviating from the original point, and the like.

Next, explanation will be made with respect to the system output information of the separation unit (HPLC) 122. The system output information of the separation unit (HPLC) 122 includes errors in the pump cylinder position and the valve position owing to abnormality in the position sensor, which occurs during driving of the pump cylinder, the injection valve, the auto purge valve, the input side stream select valve 1225, the output side stream select valve 1226, and the like. Each of the pump units 1222a, 1223a, 1224a is provided with the pump pressure sensor for monitoring the pressure in the flow passage for detecting pressure abnormality, for example, clogging in the flow passage, leakage, and the like. The system reagent unit 1221 has the position sensor for detecting the position at which abnormality in the lifter has occurred. The weight sensor is provided at a lower part of each reagent bottle mounted on the system reagent unit 1221, and monitors each amount of reagent in the respective reagent bottles. If the amount of reagent becomes smaller than the specified value, the weight sensor detects insufficiency of the reagent. The thermistor senses each surface temperature of the heater, the heat block, and the separation column. When the temperature is equal to or higher than the threshold value, temperature abnormality is detected. A column position error is caused by abnormality in the position sensor for detecting the position of the column cartridge in the column oven. Fluctuation in the syringe pressure during driving of the syringe for aspiration/discharging of the sample is monitored. Abnormality in the pressure results in clogging abnormality in the syringe, and leakage abnormality.

Next, explanation will be made with respect to the system output information of detection unit (MS) 123. The electro-spray ionizing method (ESI) is implemented for the ion source unit 1231 of the detection unit (MS) 123 to apply high-temperature, high-voltage, high current to the liquid feed passage from the separation unit (HPLC) 122 while feeding liquid through the piping with diameter of several hundreds of µm in the solution filled in the ion source unit 1231. The probe piping of double piping type has its center fed with solution. Nitrogen gas is sprayed into the piping at the outer side, and sprayed to the outside of the piping to improve the ionization efficiency. Each of the temperature, voltage value, and the current value is monitored. When each value becomes equal to or larger than the threshold value, abnormalities in the temperature, voltage value, and current value are detected. The flow passage of the nitrogen gas is monitored. Abnormality is detected upon deviation from the threshold value.

A triple-quadrupole type mass spectrometer is used for the mass spectrometer 1232, and includes a filter Q0 for converging ions ionized in the ion source to separate impurities, a filter Q1 as an ion filter for adding only the mass number of the specific m/z, a filter Q2 for generating a fragment ion through collision of gas and ions, a filter Q3 for adding only the specific fragment ion, and the detection unit. In the state where the inrush voltage, the internal voltage, and the voltage at the outlet are monitored, if the voltage and current at the inner side of the filters Q0, Q1, Q2, Q3 deviate from the respective threshold values, abnormality is detected.

### <System alarm>

Determination items to be implemented by the system alarm determination unit 113 will be described. The system alarm database 111 applies a system alarm to a determination item determined as being abnormal.
1) Abnormality in position sensor of sample probe 1213
2) Abnormality in position sensor of reagent dispensing unit 1214b
3) Abnormality in position sensor of washing probe 1216
4) Abnormality in pressure sensor of sample probe 1213
5) Abnormality in pressure sensor of reagent dispensing unit 1214b
6) Abnormality in pressure sensor of washing probe 1216
7) Abnormality in temperature sensor of reaction disk unit 1212
8) Abnormality in position sensor of sample transport unit 1211
9) Abnormality in position sensor of reaction disk unit 1212
10) Abnormality in position sensor of cleaning unit 1217
11) Abnormality in position sensor of separation unit transport unit 1218
12) Abnormality in position sensor of pump cylinder position
13) Abnormality in position sensor of injection valve
14) Abnormality in position sensor of auto purge valve
15) Abnormality in position sensor of input side stream select valve 1225
16) Abnormality in position sensor of output side stream select valve 1226
17) Abnormality in pressure sensor of pump and flow passage
18) Abnormality in position sensor of system reagent unit 1221 and lifter
19) Weight sensor of system reagent unit 1221
20) Abnormality in pressure sensor of syringe pressure sensor
21) Abnormality in temperature of column oven and heater
22) Abnormality in temperature of column oven and heat block
23) Abnormality in temperature of column oven and separation column surface
24) Abnormality in temperature of ion source
25) Abnormality in voltage of ion source
26) Abnormality in current of ion source
27) Abnormality in Q0 (inrush, internal, outlet) voltage/current
28) Abnormality in Q1 (inrush, internal, outlet) voltage/current
29) Abnormality in Q2 (inrush, internal, outlet) voltage/current
30) Abnormality in Q3 (inrush, internal, outlet) voltage/current

The determination item and the determination method are not limited to those described above.

The control device 110 acquires the system output information with time, and displays the temporal change system output information on a not shown display device. A graph is displayed on the display screen of the display device by plotting the temporal change information on the horizontal axis, and the system output information on the vertical axis. For example, the vertical axis indicates the ratio of deviation derived from comparison between the normal system alarm database 112 and the system output information.

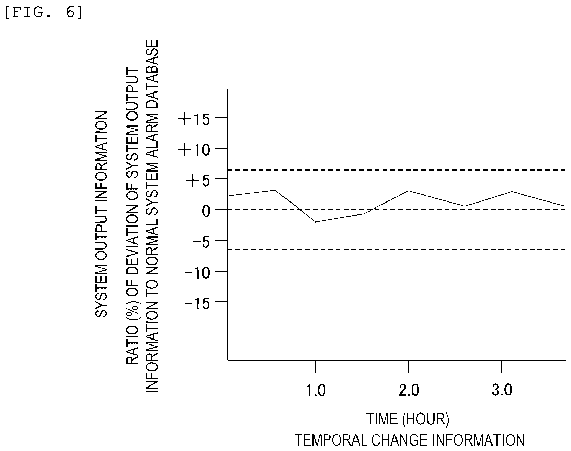

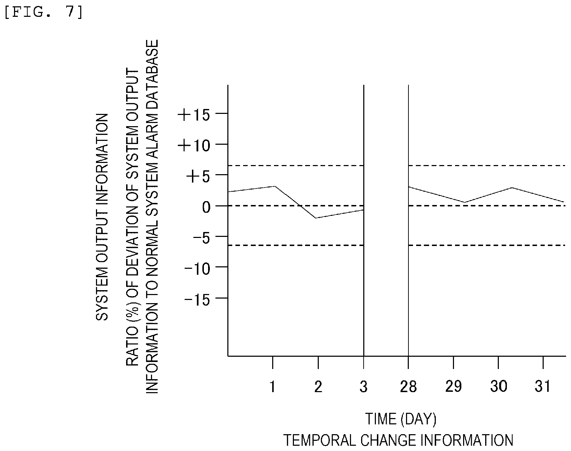

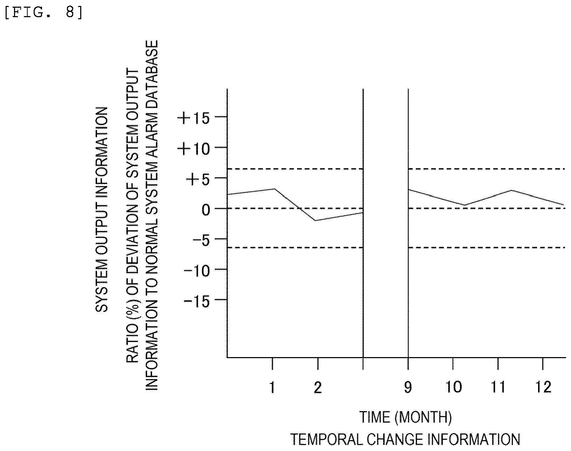

FIG. 6 to FIG. 8 are diagram showing an example of the display screen displayed on the display device. FIG. 6 represents a relationship between the temporal change information and the system output information, having the horizontal axis (time axis) set in the unit of an hour. FIG. 7 represents the relationship having the horizontal axis (time axis) set in the unit of a day. Fig. 8 represents the relationship having the horizontal axis (time axis) set in the unit of a month. FIG. 6 to FIG. 8 represent exemplary cases of abnormality in the syringe pressure sensor. The plot interval on the horizontal axis may be displayed with the whole data, data in the unit of a day, and data in the unit of a month so that the state of the syringe pressure sensor can be confirmed.

In the case of the syringe pressure sensor, the deviation of the system output information accounting for ± 20% in comparison with the normal system alarm database 112 corresponds to a threshold value in the normal range. If the value deviates from the threshold value, the system output information is determined as an abnormal value, and stored in the system alarm database 111. The system alarm is generated to allow the user to respond in accordance with the processing method to be generated concurrently with the system alarm. Another system output information can be displayed on the screen by plotting the deviation ratio of the system output information in comparison with the normal system alarm database 112 on the vertical axis, and the temporal change information on the horizontal axis. This allows grasping of the temporal change in each state of the respective pieces of the system output information.

In the predictive diagnosis performed by the generally employed automatic analyzer, abnormality is detected by comparing the system output information value of each sensor at a certain time with the normal system alarm database. Based on the temporal change information, the component is timely adjusted or replaced to reduce the downtime of the device, caused by the device failure.

In the embodiment, the timing for adjusting or replacing the component, and a defective point of the device may be grasped based on each information or combined information of the data alarm database 114 for storing the data output information generated by the analyzer 120 in addition to the system alarm database 111 for storing the system output information generated by the analyzer 120 so that the predictive diagnosis is performed. This makes it possible to reduce the downtime of the device, caused by the device failure.

An explanation will be specifically made about how to ensure the timing for adjusting or replacing the component, and the defective point in the device, and how to perform the predictive diagnosis.

### <A: Pressure sensor (rise), pressure sensor (pressure curve), retention time>

Each of the pump units 122a, 1223a, 1224a includes two cylinders driven to maintain the set flow rate, an auto purge valve for switching between the flow passage and liquid wasting to remove air in the flow passage, and the pump pressure sensor for monitoring the pressure in the flow passage to detect pressure abnormality in the flow passage downstream from the pump units 122a, 1223a, 1224a. The pressure sensor provided for each syringe is capable of detecting clogging in the flow passage and the component such as the valve in the subsequent stage of the pump units 122a, 1223a, 1224a. The pressure curve information is output in the gradient program for the pump liquid feeding in the "measurement preparation operation". In the former case, the threshold value is set to 80 MPa because of the risk of component deterioration or leakage when pressure resistance at the pump seal in the pump or the connection portion becomes excessive. In excess of the threshold value, the system alarm is generated to stop the device. In the latter case, when the pressure curve is different from the one in the normal state, for example, the quick rise in the pressure curve, it is suspected that clogging is started in the flow passage, the connection portion, or the column body. In the case of the rise delay, it is suspected that leakage occurs owing to deterioration in the flow passage, the connection portion, or the column (dead volume). If the static pressure value is higher than the one in the normal state, clogging caused by pressure is suspected. If the static pressure value is low, leakage is suspected. In this case, a data alarm is generated.

Focusing on the retention time of the internal standard substance, if the retention time is shorter than the one in the normal condition, the defective liquid feeding function of the pump is suspected (flow rate higher than the set value). If the retention time is longer than the one in the normal condition, it is suspected that the liquid feeding function of the pump is defective (flow rate lower than the set value), and the flow passage, the connection portion, or the column is deteriorated (dead volume). In this case, the data alarm is generated. In this case, the data alarm is generated.

The pump pressure can be possibly correlated with the retention time. In such a case, the cause of the defect can be easily identified. If the system sensor information of the pressure sensor value is only extracted, the user is allowed to recognize abnormality in the device. In response to the abnormality, however, it is difficult for the user to locate the specific point. Thus, the user has to ask the service person to take action against the abnormality in the end, resulting in prolonged downtime of the device. Extraction of the information in association with the data alarm of the retention time information and the pressure curve information simplifies the coping method. Extraction of the temporal change information of overpressure as the system alarm information, and the retention time and the pressure curve information as the data alarm information allows grasping of a daily state of the device.

Specifically, estimation will be made as described below.
1) If the pressure suddenly changes, and the peak of the internal standard substance cannot be detected, clogging in the flow passage can be highly estimated.
2) If both the pressure curve information and the retention time are regularly changing with time, deterioration (including clogging) of the column can be highly estimated.

In the case 1) described above, as clogging in the flow passage can be highly estimated, the user implements the maintenance menu to estimate the clogged portion caused by pressure. After locating the clogged portion caused by pressure, the item which can be handled by the user is implemented, for example, replacement of the probe of the ion source, or the component. Otherwise the service person implements the component replacement. In the case 2) described above, the user implements replacement of the column as the maintenance menu. As the temporal change information has been acquired, the information indicating that the pressure curve information and the retention time are changing with time is displayed on the GUI to clearly notify the user of such information.

### <B: S/N (ratio of peak intensity/background), current value applied to probe of ion source>

The solution which contains the internal standard substance is fed from the separation unit (HPLC) 122, and applied with high temperature, high voltage, high current in the ion source for ionization. The solution which contains the internal standard substance includes such salt as a formic acid, ammonium acetate, ammonium, and the like. In the course of ionization, the ammonium acetate salt as a volatile substance, and a contaminant may be precipitated. The contaminant is expected to be originated from a solvent of the HPLC, the component constituting the flow passage, especially, plastic member, and the component concentrated in the separation column. The probe of the ion source is regularly cleaned with a cleaning solution to reduce the risk of precipitation. Long-term continuous use may cause precipitation of salts. In this case, precipitation of the salt and the contaminant on the probe surface of the ion source, or the counter plate at the inlet of the ion source prevents the applied voltage from reaching the set value. Under the influence of the precipitated salt and contaminant, the signal is detected, which is originated from the salt having the same m/z as that of the internal standard substance output from the detection unit (MS), and the contaminant. The peak of the internal standard substance is concentrated in the separation columns 1222c, 1223c, 1224c of the separation unit (HPLC) 122. In the case of the same HPLC gradient program, the elution timing from the separation columns 1222c, 1223c, 1224c is kept unchanged. In other words, the peak of the internal standard substance is detected within the fixed retention time. Under the influence of the precipitated salt and contaminant, the peak of the internal standard substance undergoes obstruction of ionization. The resultant peak intensity becomes lower than the intrinsic peak intensity. The signal originated from the salt having the same m/z as that of the internal standard substance and the contaminant, which has been output from the detection unit (MS) is always detected in a constant amount irrespective of the retention time in the form of the constant amount of background peaks. Extraction of the temporal change information of the S/N (ratio of peak intensity/background) allows grasping of the daily state of the device.

The S/N (ratio of peak intensity/background) can be possibly correlated with the voltage of the ion source. In such a case, the cause of the defect can be easily identified. Extraction of only the system sensor information of the voltage value of the ion source as usual allows the user to recognize abnormality in the voltage of the ion source. In response to the abnormality, however, it is difficult for the user to locate the specific point. Thus, the user has to ask the service person to take action against the abnormality in the end, resulting in prolonged downtime of the device. Extraction of the information in association with the data alarm of the information on the correlation between the S/N (ratio of peak intensity/background) and the voltage of the ion source simplifies identification of the coping method. Extraction of the temporal change information of the one on the correlation among the overpressure as the system alarm information, the S/N (ratio of peak intensity/background) as the data alarm information, and the voltage of the ion source allows grasping of a daily state of the device in further detail.

Specifically, determination will be made as described below.
1) If the pressure of the ion source suddenly changes, and the peak of the internal standard substance cannot be detected, defects of the sensor by itself for applying voltage to the ion source, and the component by itself for monitoring the voltage can be highly estimated.
2) If the information on the correlation between the S/N (ratio of peak intensity/background) and the ion source voltage changes with time, it can be determined that contamination of the probe of the ion source, or the curtain plate at the inlet of the ion source is a highly possible cause.

In the case 1) as describe above, as it can be estimated with high possibility that the defect has occurred in the sensor by itself for applying voltage to the ion source, and the component by itself for monitoring the voltage, the service person is expected to replace the component. In the case 2) as describe above, the user implements the maintenance menu for removing contaminants of the probe of the ion source, or the curtain plate at the inlet of the ion source. Comparison of the voltage value of the ion source between before and after the maintenance, and comparison of the S/N (ratio of peak intensity/background) between before and after the maintenance allow grasping of the state how the contamination source has been removed.

### <C: Pressure value of syringe pressure sensor, and peak intensity (area) of internal standard substance>

Each of the sample introduction units 1222b, 1223b, 1224b includes a syringe for aspiration/discharging of the sample, and a syringe pressure sensor for monitoring the aspiration/discharging state of the syringe. In the case of insufficient removal of the viscous component of the sample, fibrin, for example, resulting from magnetic beads remaining in the processing solution during preprocessing by the preprocessing unit (SP) 121, the pressure value output from the pressure sensor upon aspiration/discharging of the syringe is compared with the normal system alarm for the sample introduction. It is then determined that abnormality has occurred. In this case, the system alarm is generated, and displayed on the GUI to notify the user of the information. Although the value is not determined as being abnormal, there may be the case of extracting the regular temporal change information indicating that the peak of the pressure value is changing stepwise.

Meanwhile, as a result of stepwise acquisition of the regular temporal change information of the peak intensity (area) of the internal standard substance, and comparison of the peak intensity (area) with the normal data alarm, it may be determined that abnormality has occurred. In this case, the data alarm is generated, and displayed on the GUI to notify the user of the information. Although the value is not determined as being abnormal, there may be the case of extracting the regular temporal change information indicating that the peak intensity (area) is changing stepwise.

Specifically, determination will be made as described below.
1) If the pressure value output from the syringe pressure sensor suddenly changes, and the peak intensity (area) of the internal standard substance cannot be detected, defects of the pressure sensor by itself can be highly determined.
2) If the information on correlation between the syringe pressure sensor and the peak intensity (area) of the internal standard substance regularly changes with time, it can be determined that clogging owing to the contaminated flow passage constituting the sample introduction units 1222b, 1223b, 1224b is a highly possible cause.

In the case 1) as described above, as it can be determined with high possibility that the defect has occurred in the syringe pressure sensor, the service person is allowed to replace the component. In the case 2) as described above, the user implements the maintenance item for cleaning the sample introduction units 1222b, 1223b, 1224b from the maintenance menu. The user then checks the device, and measures the internal standard substance while monitoring the value of the syringe pressure sensor. The user compares the information on the peak intensity (area) of the internal standard substance with the normal value for restoring the state of the device.

### <D: Weight sensor value (system reagent unit), pressure sensor (pressure curve), retention time>

Each of the sample introduction units 1222b, 1223b, 1224b includes a syringe for aspiration/discharging of the sample, and a syringe pressure sensor for monitoring the aspiration/discharging state of the syringe. The weight sensor is provided at the lower part of each reagent bottle mounted on the system reagent unit 1221. The weight sensor monitors each amount of reagent in the respective reagent bottles. If the amount of reagent becomes smaller than the specified value, the weight sensor detects insufficiency of the reagent. When aspiring/discharging the sample, the weight sensor detects insufficiency of the reagent in the reagent bottle. Based on a threshold value of insufficient reagent in the reagent bottle defined with reference to the residual value of the reagent bottle, specifically, equal to or lower than 20%, the system alarm of "warning" is generated. As the residual value equal to or lower than 10% influences the measurement result, the data alarm is generated. Because of residual reagent in the piping arranged from the reagent bottle to the pump passage, there may be the case that several measurement results obtained from generation of the data alarm are unaffected. In other words, even if the residual value of the solvent in the reagent bottle becomes equal to or lower than 10%, the measurement results may be unaffected so long as the solvent remains downstream from the pump.

Each of the pump units 1222a, 1223a, 1224a includes the pump pressure sensor for monitoring the pressure in the flow passage to detect the pressure abnormality in the flow passage downstream from the pump units 1222a, 1223a, 1224a. The pressure curve information is output in the gradient program of the pump liquid feeding in the "measurement preparation operation". The threshold value is set to 80 MPa because of the risk of component deterioration or leakage when the pressure resistance at the pump seal in the pump or the connection portion becomes excessive. In excess of the threshold value, the system alarm is generated to stop the device. When the pressure curve is different from the one in the normal state, and the rise in the pressure curve delays, it is suspected that the solvent downstream from the pump is insufficient because of insufficient reagent in the reagent bottle. Focusing on the retention time of the internal standard substance, if the retention time is long, it is suspected that the solvent downstream from the pump is insufficient because of insufficient reagent in the reagent bottle.

As described above, the data alarm is not generated to the item with respect to the relevant sample onward when the output value of the weight sensor (system reagent unit) becomes equal to or lower than 10%. In the state where the pressure curve exhibits the behavior different from the one in the normal state, and the retention time is longer than the threshold value, it is suspected that the solvent downstream from the pump is insufficient owing to insufficiency of the reagent in the reagent bottle. Accordingly, the data alarm is generated to the item with respect to the relevant sample onward.

The system output information and the data output information have been described. Acquisition of the data output information taking the system output information and the internal standard substance as indexes allows grasping of the abnormal state of the device without performing the calibration measurement, the QC (Quality Control) measurement, and the sample measurement in the "main measurement operation". Specifically, before performing the "main measurement operation", the device is checked at a predetermined time interval in the "pre-measurement preparation" to be performed by the user or the service person for selecting and implementing the maintenance item. It is preferable to check the device at start-up every morning, and analyze the temporal change information of the data alarm information, taking the system alarm output in the device check, and the internal standard substance as indexes. This makes it possible to perform the predictive diagnosis of the device without wasting the calibration reagent, the QC reagent, and the sample.

### <Supplementary Description>

The present invention is not limited to the embodiment, and may be variously modified and combined without departing from the scope of the present invention. The present invention is not necessarily limited to be configured to have all the structures as described above, and may be configured to have the structure partially omitted. The respective structures, functions and the like may be implemented by designing those elements partially or entirely using the integrated circuit, for example. The respective structures, functions and the like may be implemented by software through which the processor interprets and executes the program for actualizing the respective functions.

### Reference Sign List

- 100: automatic analyzer
- 100: clinical diagnosis system
- 101: sample container rack
- 101a: sample container
- 110: control device
- 111: system alarm database
- 112: normal system alarm database
- 113: system alarm determination unit
- 114: data alarm database
- 115: normal data alarm database
- 116: data alarm determination unit
- 120: analyzer
- 121: preprocessing unit (SP)
- 122: separation unit (HPLC)
- 122a: pump unit
- 123: detection unit (MS)
- 124: mass spectrometry analysis processing unit
- 130: external computer
- 131: integration platform
- 1211: sample transport unit
- 1212: reaction disk unit
- 1212a: reaction container
- 1213: sample probe
- 1214: reagent disk unit
- 1214a: reagent container
- 1214b: reagent dispensing unit
- 1215: reagent container holding unit
- 1215a: reagent probe
- 1216: washing probe
- 1217: cleaning unit
- 1218: separation unit transport unit
- 1221: system reagent unit
- 1222: HPLC1 stream
- 1222a: pump unit
- 1222b: sample introduction unit
- 1222c: separation column
- 1223: HPLC2 stream
- 1223a: pump unit
- 1223b: sample introduction unit
- 1223c: separation column
- 1224: HPLC3 stream
- 1224a: pump unit
- 1224b: sample introduction unit
- 1224c: separation column
- 1225: input side stream select valve
- 1226: output side stream select valve
- 1231: ion source unit
- 1232: mass spectrometer

## Claims

1. An automatic analyzer, comprising:
an analyzer including a separation unit configured to perform separation of a sample to be measured subjected to a pretreatment, and a detection unit configured to detect a substance to be measured of the sample separated by the separation unit; and
a control device configured to control an operation of the analyzer, wherein
the control device includes:
a data alarm database that stores data output information including at least one item generated based on a detection result of the sample of the analyzer;
a normal data alarm database that stores predetermined reference data output information to determine whether the data output information is in a normal state or in an abnormal state;
a data alarm determination unit configured to compare the data output information of the data alarm database with the reference data output information of the normal data alarm database and apply a data alarm to an item of the data output information of the data alarm database determined to be in an abnormal state;
a system alarm database that stores system output information including at least one item generated based on a detection result from a sensor provided in the analyzer;
a normal system alarm database that stores predetermined reference system output information to determine whether the system output information is in a normal state or in an abnormal state; and
a system alarm determination unit configured to compare the system output information of the system alarm database with the reference system output information of the normal system alarm database and apply a data alarm to an item of the data output information of the system alarm database determined to be in an abnormal state, and
the control device is configured to execute a predictive diagnosis of a failure state of the automatic analyzer based on a temporal change of the data output information that is obtained by the analyzer using an internal standard substance as the sample and is stored in the data alarm database and a temporal change of the system output information of the system alarm database.

2. The automatic analyzer according to claim 1, wherein
the system output information stored in the system alarm database by the control device includes at least one of:
a peak area ratio of the substance to be measured and the internal standard substance;
a peak intensity ratio of the substance to be measured and the internal standard substance;
a peak area of the substance to be measured;
a peak intensity of the substance to be measured;
a retention time of the substance to be measured;
a peak width of the substance to be measured;
a full width at half maximum of the substance to be measured;
an S/N ratio which is a ratio of the peak intensity to a background of the substance to be measured;
a leading factor of the substance to be measured;
a tailing factor of the substance to be measured;
a peak area of the internal standard substance;
a peak intensity of the internal standard substance;
a retention time of the internal standard substance;
a peak width of the internal standard substance;
a full width at half maximum of the internal standard substance;
an S/N ratio which is a ratio of the peak intensity to a background of the internal standard substance;
a leading factor of the internal standard substance; and
a tailing factor of the internal standard substance.

3. The automatic analyzer according to claim 1, wherein
the data output information stored in the data alarm database by the control device includes at least one of:
a signal value of the internal standard substance;
an identification check (the retention time, the peak width, the full width at half maximum, and the S/N (the ratio of the peak intensity to the background) of the internal standard substance) of the internal standard substance;
a signal value of the substance to be measured;
an identification check (the retention time, the peak width, the full width at half maximum, and the S/N (the ratio of the peak intensity to the background) of the internal standard substance) of the substance to be measured;
a HPLC performance check (a HPLC pressure curve, an ESI probe current, and the peak width, a peak symmetry, and the retention time of the internal standard substance);
an MS performance check (a peak parameter, a background intensity, a noise value, and contamination information);
calibration curve information; and
a QC check.

4. The automatic analyzer according to claim 1, wherein
a predictive diagnosis is executed using the internal standard substance as an index without using any of a calibration reagent, a QC reagent, and a sample in a pre-measurement preparation step of a user or a service person selecting and executing a maintenance item after a start-up step of checking communication between the analyzer and the control device and checking a state of each sensor and each consumable after power is turned on.

5. A predictive diagnosis method for a failure state of an automatic analyzer including a separation unit configured to perform separation of a sample to be measured subjected to a pretreatment, a detection unit configured to detect a substance to be measured of the sample separated by the separation unit, and a control device configured to control an operation of the analyzer, the predictive diagnosis method comprising:
a step of storing, in a data alarm database, data output information including at least one item generated based on a detection result of the sample of the analyzer;
a step of comparing the data output information of the data alarm database with predetermined reference data output information, and applying a data alarm to an item of the data output information of the data alarm database determined to be in an abnormal state;
a step of storing, in a system alarm database, system output information including at least one item generated based on a detection result from a sensor provided in the analyzer;
a step of comparing the system output information of the system alarm database with predetermined reference system output information, and applying a data alarm to an item of the data output information of the system alarm database determined to be in an abnormal state; and
a step of executing a predictive diagnosis of a failure state based on a temporal change of the data output information that is obtained by the analyzer using an internal standard substance as the sample and is stored in the data alarm database and a temporal change of the system output information of the system alarm database.
